# EUROPEAN PATENT SPECIFICATION

(11) **EP 4 509 073 B1**
(45) Date of publication and mention of the grant of the patent: **09.12.2026**
(21) Application number: 23722609.7
(22) Date of filing: 11.04.2023
(51) Int. Cl.: A61B 17/3207, A61B 10/02, A61B 10/04, A61B 17/00, A61B 17/32

(54) **PORTABLE ACTUATOR DEVICE OF A MEDICAL APPARATUS FOR TAKING BIOPSIES AND EXTRACTING TISSUE BY HYSTEROSCOPY**
TRAGBARE BETÄTIGUNGSVORRICHTUNG EINER MEDIZINISCHEN VORRICHTUNG ZUR ENTNAHME VON BIOPSIEN UND ZUR EXTRAKTION VON GEWEBE DURCH HYSTEROSKOPIE
DISPOSITIF D'ACTIONNEMENT PORTATIF D'UN INSTRUMENT MÉDICAL POUR RÉALISER DES BIOPSIES ET PRÉLEVER DES TISSUS PAR HYSTÉROSCOPIE

(30) Priority: 11.04.2022 ES 202230328
(43) Date of publication of application: 19.02.2025
(73) Proprietor: Alonso Pacheco, Luis, 29630 Benalmádena (Malaga) (ES); Haimovich Segal, Sergio Mario, 29630 Benalmádena (Malaga) (ES); Carugno Matute, José Antonio, 29630 Benalmádena (Malaga) (ES); Di Spiezio Sardo, Attilio, 29630 Benalmádena (Malaga) (ES); Rosado Rios, Juan, 29631 Benalmádena (Malaga) (ES)
(72) Inventor: Alonso Pacheco, Luis, 29630 Benalmádena (Malaga) (ES); Haimovich Segal, Sergio Mario, 29630 Benalmádena (Malaga) (ES); Carugno Matute, José Antonio, 29630 Benalmádena (Malaga) (ES); Di Spiezio Sardo, Attilio, 29630 Benalmádena (Malaga) (ES); Rosado Rios, Juan, 29631 Benalmádena (Malaga) (ES)
(74) Representative: Isern Patentes y Marcas S.L.
(86) International application number: PCT/ES2023/070230
(87) International publication number: WO 2023/198950

(56) References cited:
- WO-A1-2016/100522
- US-A- 4 541 423
- US-A1- 2016 066 915
- US-A1- 2018 360 495
- US-A1- 2019 008 538
- US-A1- 2020 297 372
- US-A1- 2021 169 476

## Description

### OBJECT OF THE INVENTION

The object of the present invention is to provide a portable and autonomously operating device intended for taking samples and performing biopsies by means of hysteroscopy in the uterus of a patient.

### TECHNICAL FIELD

The invention belongs to the field of apparatuses in the medical sector, and particularly those used for hysteroscopy.

### BACKGROUND OF THE INVENTION

The uterus is a vitally important organ in the human species, since it is responsible for housing the embryo and promoting its development until a living being is mature enough to be able to live outside the "maternal womb", that is, outside the womb. own womb.

In fact, the uterus is a hollow organ and fetal development occurs just inside this hollow part, which is called the uterine cavity. All that pathology, all that problem that affects that hollow part, has an implication on the reproductive capacity of the uterus, for which reason the treatment of this pathology is of great importance and, currently, it is performed using the hysteroscopy technique.

Hysteroscopy consists of introducing a camera into the uterine cavity in order to be able to visualize the interior of the uterus. Therefore, it is suitable for the diagnosis of various problems that affect the cavity and can also be used, with the use of various apparatuses and specific devices, for the surgical treatment of said pathology.

This surgery has continuously evolved over the last few years thanks to technological development and the possibility of miniaturizing the instruments used. In this way, the apparatuses have evolved from simple scissors, through the use of electrosurgery to mechanical tissue resection systems (known as Hysteroscopic Tissue Renewal Systems HTRs), also called hysteroscopic morcellators.

Intrauterine morcellators have been developed in an attempt to overcome the limits of traditional hysteroscopic resectoscopy systems, such as the risks of using electrosurgical energy and thermal injuries based on mechanical action not dependent on electrical current.

Besides, the ability to cut and aspirate tissue simultaneously optimizes tissue removal and, most importantly, avoids the need for additional apparatus.

Thus, different devices are known, some of which are based on the use of only mechanical energy (rotation of the blade) and aspiration of the fragments, while other devices or systems perform bipolar radiofrequency cutting.

All these previously mentioned systems present a similar design in such a way that they are composed of a generating unit managed by specific software, an activation pedal, a specific hysteroscope and a cutting blade mechanism.

The mechanism of HTRs systems is very simple. Once the window of the cutting blade is placed in close contact with the patient's regions to be excised, the tissue is simultaneously cut and aspirated, thus improving visibility for the surgeon and reducing the need for multiple removals and insertions. of the device from the uterine cavity with the risk of causing some damage to said region of the patient. However, in practice it has been observed that this mechanism presents a series of drawbacks, such as the fact that each system uses its own generating unit, its own hysteroscope, they have variable diameters that generally require dilating the inlet part. to the uterus prior to the insertion of the hysteroscope, and they are for single use, so they imply a considerable economic cost for the health center or the public administration.

Tissue extraction apparatus with a manual drive system with probes of different diameters are also known. Once used, these types of devices are completely discarded, so they can only be used on a single time.

It is known from prior art document US 2020/297372 disclosing a medical instrument for resecting tissue wherein common members are part of the preamble of claim 1. Also, document US 4541423 discloses a drilling apparatus for attachment to a rotary motor includes a flexible shaft.

Furthermore, the applicant is currently unaware of an invention that has all the features described in this specification.

### DESCRIPTION OF THE INVENTION

The present invention has been developed with the aim of providing a portable actuator device that is configured as a novelty within the field of application and solves the previously mentioned drawbacks, further contributing other additional advantages that will be obvious from the description below.

An object of the present invention is therefore to provide a portable actuator device of a medical apparatus for taking biopsies and extracting tissue by hysteroscopy, as defined in claim 1.

Given these features, an easily portable device which allows the movement of the cutting means to be generated is obtained, reducing the involuntary movements that take place when the cutting means are activated by a manual generator, thus achieving a more uniform and accurate movement, which has a positive impact on the accuracy of the surgical operation.

The fact that the portable device can be used in any standard hysteroscope with an operating channel and can be detached from the probe provided with the cutting means allows a cost reduction as it does not require the disposal of an entire instrument for hysteroscopy.

Also, the fact that it incorporates an autonomous power supply system avoids the use of connection cables during the use of the apparatus that could otherwise hinder any movement during a surgery, so the advantages which this device has are evident in comparison to the so-called intrauterine morcellators in which the control unit (of dimensions that are not portable in a single hand) is coupled to the probe by means of cables.

According to the invention, the fastening means comprise an extension that protrudes from one end of the main casing, which is externally provided with a threaded portion, a connector member configured to be threaded to the threaded portion acting as a pressure means configured to press on the probe, the side wall that forms the extension having a plurality of grooves arranged radially in such a way that they define a plurality of flexible fins intended to act by pressure on the probe. In this way, once the body of the cutting blade has been inserted, when the connector member rotates it presses the section of the engageable probe inside the extension, thus the fastening system is simple to manufacture and reliable.

Preferably, the coupling portion of the rotary drive shaft has a central portion provided with a plurality of projections that protrude radially from the central portion, projections having a flat edge that is inclined with respect to a flat edge located at the end of the central portion of the rotary drive shaft.

According to the invention, the actuator device includes sensor means configured to detect the position of the probe in the extension, the sensor means being linked (by electrical connection) with a programmable control unit present inside the main casing. In this way, the control unit can inform the surgeon that the probe is correctly attached without the risk of it being released inadvertently during use of the medical apparatus for hysteroscopy practice. In addition, the arrangement of the sensor means allows to automatically close the cutting window located at the end of the probe with the cutting blade, avoiding unwanted cuts by the sharp sides of the cutting window and avoiding the suction of liquids when not be required.

The sensor means comprise a Hall sensor located inside the extension.

According to another feature of the actuator device of the invention, the user interface may comprise a plurality of electromechanical pushbuttons located in the main casing, which act on the cutting means provided in the probe.

Additionally, the user interface can comprise a display screen located in the main casing, that provides visual information to the user or surgeon in a simple and quick way.

It is another object of the invention to provide a medical apparatus for the practice of hysteroscopy, comprising a probe formed by an elongated conduit that includes an interior channel and cutting means configured to act on a patient's tissue, characterized in that the cutting means are actuatable by an actuator device as above disclosed.

The described actuator device therefore represents an innovative structure with structural and constitutional features hitherto unknown for its intended purpose, reasons which, taken together with its usefulness, provide it with sufficient grounds for obtaining the requested exclusivity privilege.

Other features and advantages of the actuator device of the present invention will be evident in light of the description of a preferred, but not exclusive, embodiment which is illustrated by way of a non-limiting example in the drawings which are attached, wherein:

### BRIEF DESCRIPTION OF THE DRAWINGS

Figure 1 is a perspective view of the medical apparatus for taking biopsies and extracting tissue according to the present invention;
Figure 2 is a perspective view of the portable actuator device according to the invention;
Figure 3 is a partially exploded perspective view of the portable actuator device represented in Figure 2;
Figure 4 is a perspective view from the rear of the portable actuator device;
Figure 5 is a front perspective view of the portable actuator device of the invention;
Figure 6 is a perspective view of the probe that can be coupled to the portable actuator device;
Figure 7 is a rear perspective view of the probe represented in Figure 6;
Figure 8 is a perspective detailed view of the end of the probe where the cutting means are shown; and
Figure 9 is a partially sectioned perspective view of the area corresponding to the junction between the portable actuator device of the invention and a probe provided with cutting means.

### DESCRIPTION OF A PREFERRED EMBODIMENT

In light of the aforementioned figures, and in accordance with the adopted numbering, one may observe therein a preferred exemplary embodiment of the invention, which comprises the parts and elements indicated and described in detail below.

Furthermore, the terms first, second, third and the like in the description and in the claims are used to distinguish between similar items and not necessarily to describe a sequential or chronological order. The terms may be interchanged in appropriate circumstances and embodiments of the invention may operate in sequences other than those described or illustrated herein.

Furthermore, the terms top, bottom, top, bottom, and the like in the description and claims are used for descriptive purposes and not necessarily to describe relative positions.

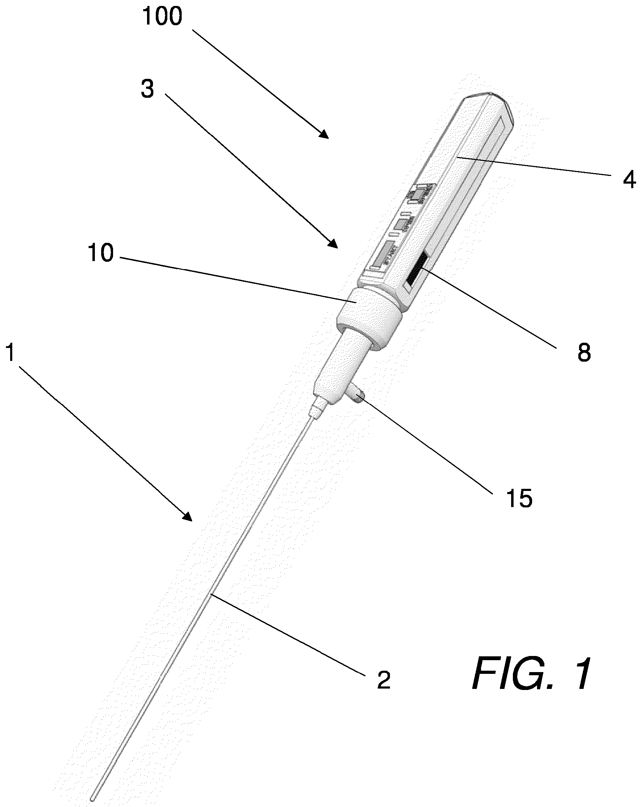

As can be seen in Figure 1, a medical apparatus for the practice of hysteroscopy, generally indicated by numeral reference (100), essentially comprises two main parts: a probe (1) formed by an elongated conduit (2) that includes an interior channel and cutting means (disclosed below) configured to act on a patient's tissue, and an actuator device, generally indicated by numeral reference (3), which is configured to act on the cutting means.

Going into greater detail about portable actuator device (3) for medical apparatus (100), it comprises a processor module comprising a main casing (4) made up of any suitable material, such as a plastic material, and with a size that is easily graspable or handle by the surgeon. To note that the portable actuator device (3) can be attached to an accessory in the form of a handle to facilitate the user's handling of the medical apparatus.

The actuator device is provided with fastening means (disclosed with more details below) configured for coupling releasably to a probe (1) that includes cutting means configured to act on a patient's tissue. Motorized means are also provided inside the main casing (4), provided with a rotary drive shaft (5) configured to perform a movement on the cutting means, the rotary drive shaft (5) having a coupling region intended to be coupled to the cutting means, the motorized means being actuatable through a user interface.

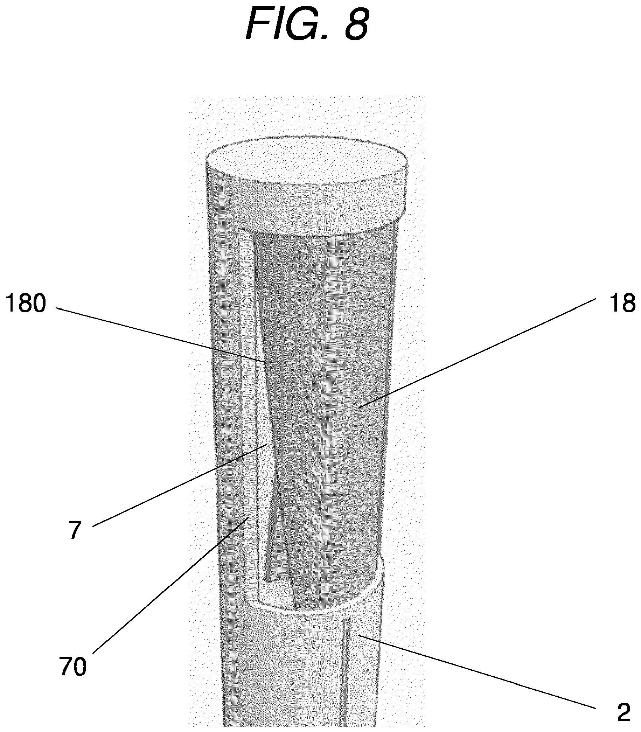

The user interface comprises a plurality of electromechanical pushbuttons (6) located in the main casing (4), also called control pushbuttons also, which allow to regulate, for example, the cutting speed of the cutting means, activate the opening and/or closing of a cutting window (7) located at the end of the probe (1), as can be seen in the figure 8, which causes the cutting blade of the cutting means to rotate up to 180º while a specific pushbutton (6) is kept pressed, allowing suction through the cutting window (7).

The user interface also has a small-sized display screen (8) located in the main casing (4) that indicates the status of the device as a whole, the speed selected for the cutting blade and the battery status.

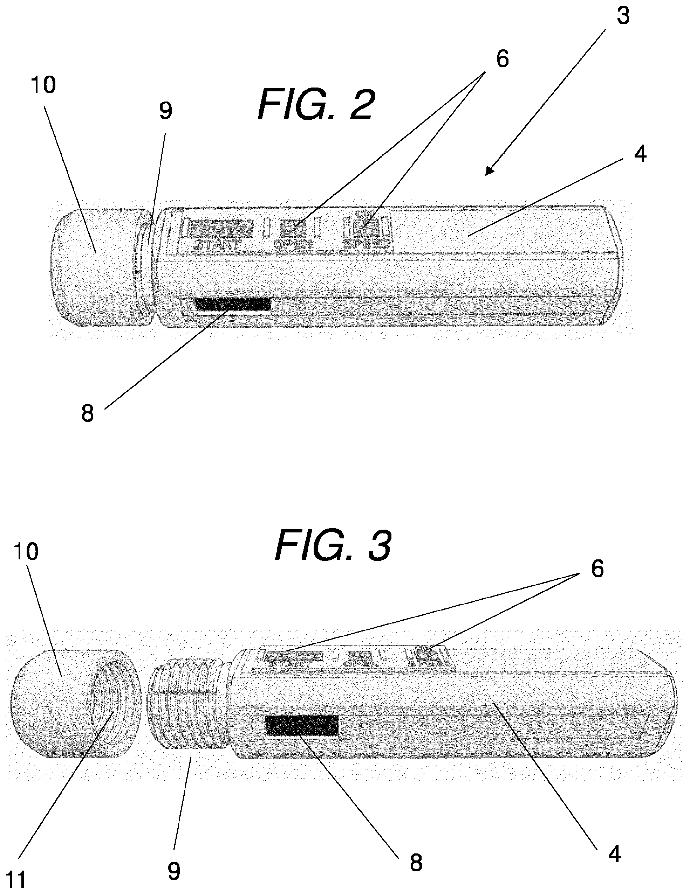

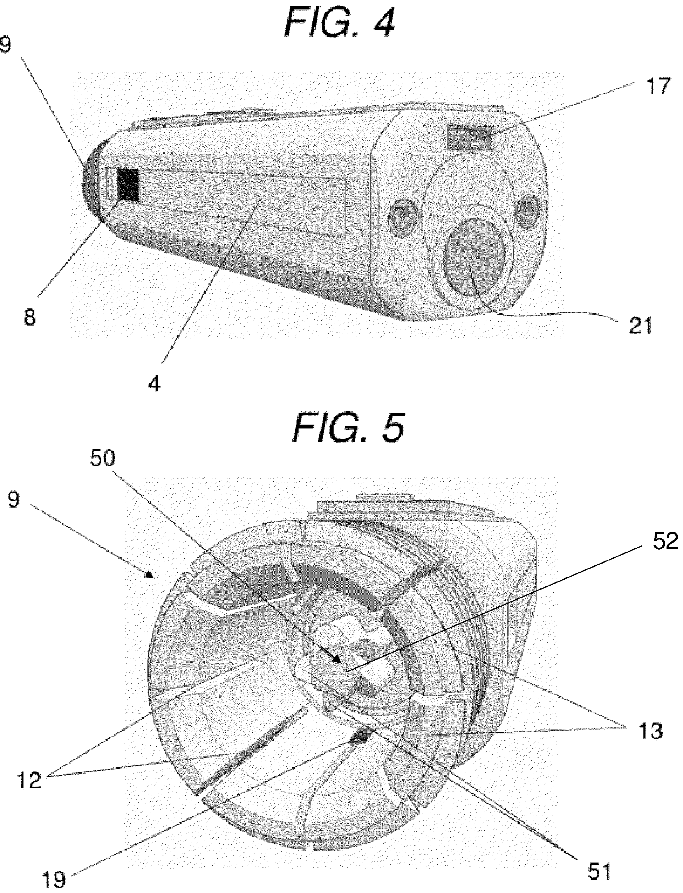

Now, as can be seen in figure 3, and particularly relating to the fastening means comprise an extension (9) that protrudes from one end of the main casing (4), which is externally provided with a threaded portion, a connector member (10) formed by a sleeve (generally cylindrical in shape) with an internal thread (11) configured to be threaded to the threaded portion acting as a pressure means configured to press on the probe (1). As shown in the figure 5, the side wall that forms the extension (9) having a plurality of grooves (12) arranged radially in such a way that they define a plurality of flexible fins (13) intended to act by pressure on the probe (1).

In greater detail, the coupling region of the rotary drive shaft (5) has a central portion (50) provided with a plurality of projections (51) which have a flower petal shape, which protrude radially from the central portion, projections (51) having a flat edge that is inclined with respect to a flat edge located at the end of the central portion of the rotary drive shaft.

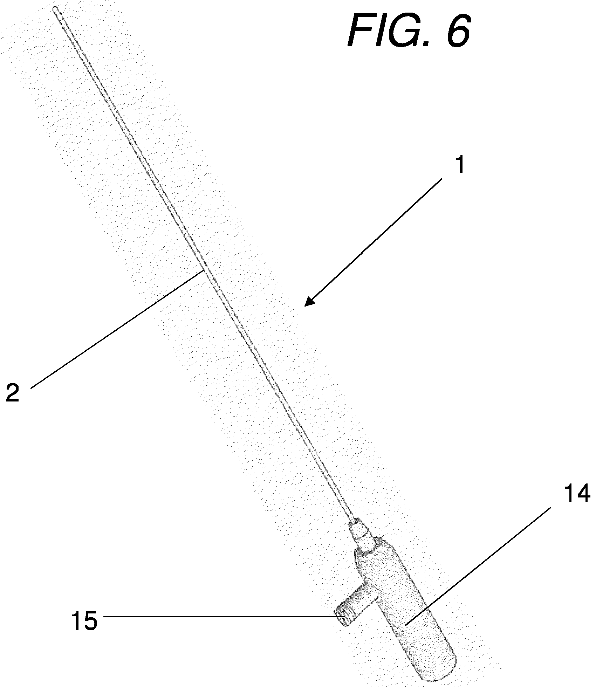

Returning again to the probe, as can be seen in figure 6, the elongated conduit (2) ends at one end in a widened portion (14). This widened portion (14) has an internally hollow branch (15) that extends perpendicularly, which acts as a suction tube, in such a way that it can be connected to an external suction system by means of a standard flexible conduit (not shown), thanks to the arrangement of diametrical projections that serve as fixing means for the flexible duct.

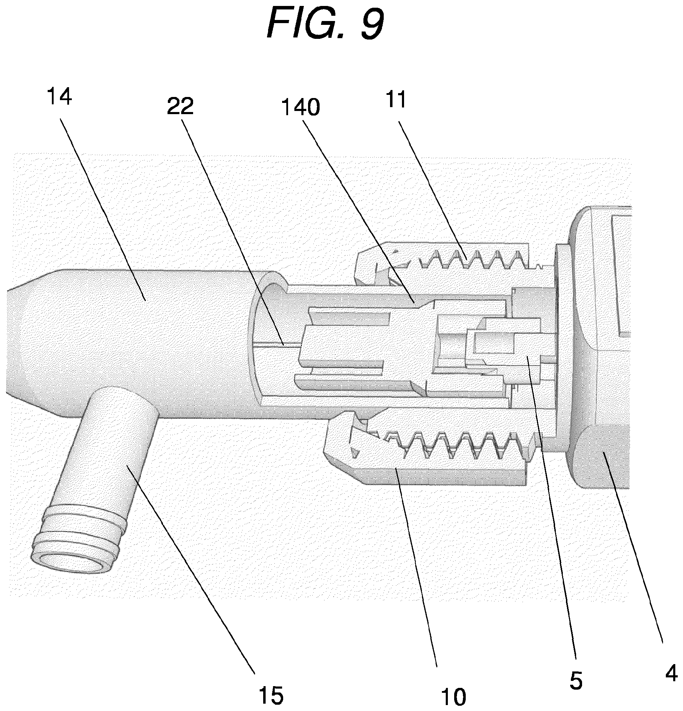

Figure 9 shows the arrangement adopted by the connector member (10) with the extension (9) when the probe (1) is coupled to the portable actuator device (3), in such a way that when the connector member (10) is fully threaded into the extension (9), said extension (9) exerts a pressure on the part (140) of the widened portion (14).

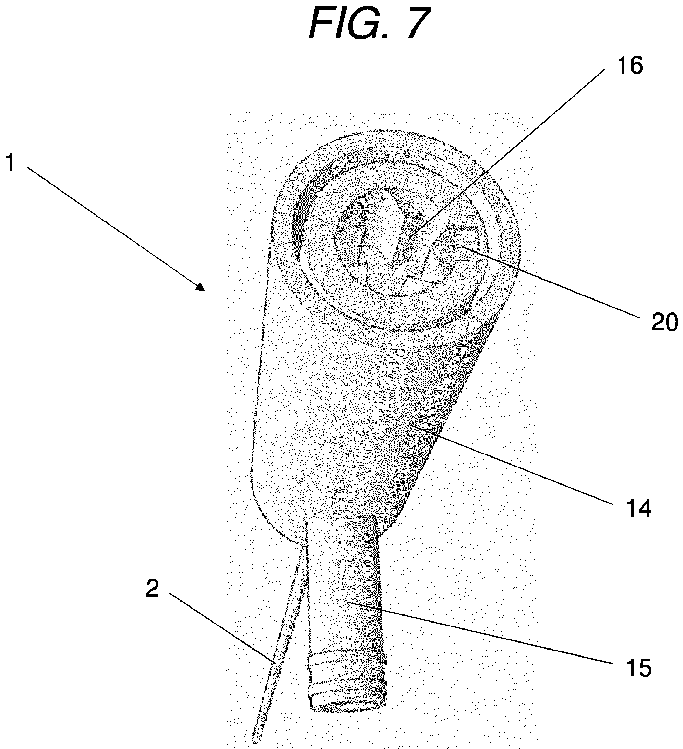

As can be seen in figure 7, the widened portion also presents a coupling portion (16), which has a complementary design with the coupling portion of the rotary drive shaft (5), to allow coupling of the coupling region of the rotary drive shaft (5).

As regards the cutting means, they comprise a cutting blade (18) located at the end of the elongated conduit (2), specifically in the cutting window (7), and which can rotate due to the provision of a drive shaft (22) (see figure 9) which runs internally along the length of the elongated conduit (2). As can be seen in figure 8, the cutting blade (18) has a cutting edge (180) that is oblique with respect to an edge (70) of the cutting window (7), which enhances the cutting action, that is, it improves the cutting precision of the tissue to be removed.

Additionally, in order to ensure that the attachment between the probe (2) and the actuator device is correct and has not been improperly positioned, sensor means configured to detect the position of the probe in the extension are provided, the sensor means being with a programmable control unit placed inside the main casing (4). These sensor means comprise a Hall sensor (19) located inside the extension. The coupling portion (16) includes a reference point (20), which consists of a recess, which is located in a complementary way to the position of the Hall sensor (19).

On the rear region of the main casing (4) there is an ON/OFF button (21) to switch on the motorized means of the portable actuator device (3), as well as a recharging connector (17) of standard type configured to charge the rechargeable battery that acts on the motorized means (electric motor).

The details, shapes, dimensions and other accessory elements, used to manufacture the portable actuator device of the invention, may be suitably replaced for others which do not depart from the scope defined by the claims which are included below.

### List of numeral references

- 1.: probe
- 2.: elongated conduit
- 3.: actuator device
- 4.: main casing
- 5.: rotary drive shaft
- 50.: central portion
- 51.: projections
- 6.: pushbuttons
- 7.: cutting window
- 70.: edge
- 8.: display screen
- 9.: extension
- 10.: connector member
- 11.: internal thread
- 12.: grooves
- 13.: flexible fins
- 14.: widened portion
- 15.: branch
- 16.: coupling portion
- 17.: recharging connector
- 18.: cutting blade
- 19.: Hall sensor
- 20.: reference point
- 21.: ON/OFF button
- 22.: drive shaft
- 100.: Medical apparatus for the practice of hysteroscopy

## Claims

1. Portable actuator device (3) of a medical apparatus (100) for taking biopsies and extracting tissue by hysteroscopy, which has a processor module, the portable actuator device comprising:
a main casing (4) provided with fastening means configured for coupling releasably to a probe (1) that includes cutting means configured to act on a patient's tissue,
a motorized means provided with a rotary drive shaft (5) configured to perform a movement on the cutting means, the rotary drive shaft (5) having a coupling portion configured to be coupled to the cutting means, the motorized means being actuatable through a user interface and by an autonomous electrical power supply system, **characterised in that** the fastening means comprise an extension (9) that protrudes from one end of the main casing (4), which is externally provided with a threaded portion, a connector member (10) configured to be threaded to the threaded portion acting as a pressure means configured to press on the probe (1), the side wall that forms the extension having a plurality of grooves arranged radially in such a way that they define a plurality of flexible fins configured to press on the probe (1), wherein the portable actuator device includes sensor means configured to detect the position of the probe (1) in the extension (9), the sensor means being linked with a programmable control unit present inside the main casing, wherein the sensor means comprise a Hall sensor located inside the extension (9).

2. Portable actuator device (3) according to claim 1, wherein the coupling region of the rotary drive shaft (5) has a central portion (50) provided with a plurality of projections (51) that protrude radially from the central portion, the projections having a flat edge that is inclined with respect to a flat edge (52) located at the end of the central portion (50) of the rotary drive shaft (5).

3. Portable actuator device (3) according to claim 1, wherein the user interface comprises a plurality of electromechanical pushbuttons (6) located in the main casing (4).

4. Portable actuator device (3) according to claim 1, wherein the user interface comprises a display screen (8) located in the main casing (4).

5. Portable actuator device (3) according to claim 1, wherein it includes a standard type recharging connector configured to charge a battery that drives the motorized means.

6. Medical apparatus (100) for the practice of hysteroscopy, comprising a probe (1) formed by an elongated conduit that includes an interior channel and cutting means configured to act on a patient's tissue, including an actuator device according to any of claims 1 to 5, wherein the cutting means are actuatable by the actuator device according to any of claims 1 to 5, and wherein the actuator device is releasably coupled to said probe.

7. Medical apparatus (100) according to claim 6, wherein the cutting means comprises a cutting blade located in a window located in the probe (1), being coupled to a drive shaft that runs along of the elongated conduit, the drive shaft being movable by the action of the rotary drive shaft provided in the drive device.

8. Medical apparatus (100) according to claim 7, wherein the cutting blade has an edge that is oblique with respect to an edge of the cutting window.

## Patentansprüche

1. Tragbare Betätigungsvorrichtung (3) eines medizinischen Geräts (100) zur Entnahme von Biopsien und zur Extraktion von Gewebe mittels Hysteroskopie, mit einem Prozessormodul, wobei die tragbare Betätigungsvorrichtung Folgendes umfasst:
ein Hauptgehäuse (4), das mit Befestigungsmitteln versehen ist, die dazu ausgelegt sind, lösbar mit einer Sonde (1) gekoppelt zu werden, die Schneidmittel beinhaltet, die dazu ausgelegt sind, auf das Gewebe eines Patienten einzuwirken,
ein motorisiertes Mittel, das mit einer rotierenden Antriebswelle (5) versehen ist, die dazu ausgelegt ist, eine Bewegung an den Schneidmitteln auszuführen, wobei die rotierende Antriebswelle (5) einen Kopplungsabschnitt aufweist, der dazu ausgelegt ist, mit den Schneidmitteln gekoppelt zu werden, wobei das motorisierte Mittel über eine Benutzerschnittstelle und durch ein autonomes elektrisches Stromversorgungssystem betätigbar ist,
**dadurch gekennzeichnet, dass** die Befestigungsmittel eine Verlängerung (9) umfassen, die von einem Ende des Hauptgehäuses (4) vorsteht und die außen mit einem Gewindeabschnitt versehen ist, sowie ein Verbindungselement (10), das dazu ausgelegt ist, auf den Gewindeabschnitt aufgeschraubt zu werden, und das als Druckmittel wirkt, das dazu ausgelegt ist, auf die Sonde (1) zu drücken, wobei die Seitenwand, die die Verlängerung bildet, eine Vielzahl von radial angeordneten Nuten aufweist, derart, dass sie eine Vielzahl von flexiblen Lamellen definieren, die dazu ausgelegt sind, auf die Sonde (1) zu drücken, wobei die tragbare Betätigungsvorrichtung Sensormittel beinhaltet, die dazu ausgelegt sind, die Position der Sonde (1) in der Verlängerung (9) zu erfassen, wobei die Sensormittel mit einer programmierbaren Steuereinheit verbunden sind, die sich im Inneren des Hauptgehäuses befindet, wobei die Sensormittel einen Hall-Sensor umfassen, der sich im Inneren der Verlängerung (9) befindet.

2. Tragbare Betätigungsvorrichtung (3) nach Anspruch 1, wobei der Kopplungsbereich der rotierenden Antriebswelle (5) einen zentralen Abschnitt (50) aufweist, der mit einer Vielzahl von Vorsprüngen (51) versehen ist, die radial von dem zentralen Abschnitt vorstehen, wobei die Vorsprünge eine flache Kante aufweisen, die in Bezug auf eine flache Kante (52) geneigt ist, die sich am Ende des zentralen Abschnitts (50) der rotierenden Antriebswelle (5) befindet.

3. Tragbare Betätigungsvorrichtung (3) nach Anspruch 1, wobei die Benutzerschnittstelle eine Vielzahl von elektromechanischen Drucktasten (6) umfasst, die im Hauptgehäuse (4) angeordnet sind.

4. Tragbare Betätigungsvorrichtung (3) nach Anspruch 1, wobei die Benutzerschnittstelle einen Anzeigebildschirm (8) umfasst, der im Hauptgehäuse (4) angeordnet ist.

5. Tragbare Betätigungsvorrichtung (3) nach Anspruch 1, wobei sie einen Ladeanschluss vom Standardtyp beinhaltet, der dazu ausgelegt ist, eine Batterie zu laden, die das motorisierte Mittel antreibt.

6. Medizinisches Gerät (100) zur Durchführung der Hysteroskopie, umfassend eine Sonde (1), die durch eine längliche Leitung gebildet ist, die einen inneren Kanal und Schneidmittel beinhaltet, die dazu ausgelegt sind, auf das Gewebe eines Patienten einzuwirken, einschließlich einer Betätigungsvorrichtung nach einem der Ansprüche 1 bis 5, wobei die Schneidmittel durch die Betätigungsvorrichtung nach einem der Ansprüche 1 bis 5 betätigbar sind und wobei die Betätigungsvorrichtung lösbar mit der genannten Sonde gekoppelt ist.

7. Medizinisches Gerät (100) nach Anspruch 6, wobei die Schneidmittel eine Schneidklinge umfassen, die sich in einem Fenster befindet, das sich in der Sonde (1) befindet, und die mit einer Antriebswelle gekoppelt ist, die entlang der länglichen Leitung verläuft, wobei die Antriebswelle durch die Wirkung der in der Antriebsvorrichtung vorgesehenen rotierenden Antriebswelle bewegbar ist.

8. Medizinisches Gerät (100) nach Anspruch 7, wobei die Schneidklinge eine Kante aufweist, die in Bezug auf eine Kante des Schneidfensters schräg ist.

## Revendications

1. Dispositif actionneur portable (3) d'un appareil médical (100) pour le prélèvement de biopsies et l'extraction de tissu par hystéroscopie, qui présente un module processeur, le dispositif actionneur portable comprenant :
un boîtier principal (4) pourvu de moyens de fixation configurés pour s'accoupler de manière amovible à une sonde (1) qui comporte des moyens de coupe configurés pour agir sur le tissu d'un patient,
un moyen motorisé pourvu d'un arbre d'entraînement rotatif (5) configuré pour effectuer un mouvement sur les moyens de coupe, l'arbre d'entraînement rotatif (5) ayant une partie d'accouplement configurée pour être accouplée aux moyens de coupe, le moyen motorisé pouvant être actionné par l'intermédiaire d'une interface utilisateur et au moyen d'un système d'alimentation électrique autonome,
**caractérisé en ce que** les moyens de fixation comprennent un prolongement (9) qui fait saillie à partir d'une extrémité du boîtier principal (4), qui est pourvu extérieurement d'une partie filetée, un élément de raccordement (10) configuré pour être vissé sur la partie filetée agissant comme moyen de pression configuré pour presser sur la sonde (1), la paroi latérale qui forme le prolongement présentant une pluralité de rainures disposées radialement de telle sorte qu'elles définissent une pluralité d'ailettes flexibles configurées pour presser sur la sonde (1), dans lequel le dispositif actionneur portable comporte des moyens de détection configurés pour détecter la position de la sonde (1) dans le prolongement (9), les moyens de détection étant reliés à une unité de commande programmable présente à l'intérieur du boîtier principal, dans lequel les moyens de détection comprennent un capteur à effet Hall situé à l'intérieur du prolongement (9).

2. Dispositif actionneur portable (3) selon la revendication 1, dans lequel la zone d'accouplement de l'arbre d'entraînement rotatif (5) présente une partie centrale (50) pourvue d'une pluralité de protubérances (51) qui font saillie radialement à partir de la partie centrale, les protubérances présentant un bord plat qui est incliné par rapport à un bord plat (52) situé à l'extrémité de la partie centrale (50) de l'arbre d'entraînement rotatif (5).

3. Dispositif actionneur portable (3) selon la revendication 1, dans lequel l'interface utilisateur comprend une pluralité de boutons-poussoirs électromécaniques (6) situés dans le boîtier principal (4).

4. Dispositif actionneur portable (3) selon la revendication 1, dans lequel l'interface utilisateur comprend un écran d'affichage (8) situé dans le boîtier principal (4).

5. Dispositif actionneur portable (3) selon la revendication 1, dans lequel il comporte un connecteur de recharge de type standard configuré pour charger une batterie qui entraîne le moyen motorisé.

6. Appareil médical (100) pour la pratique de l'hystéroscopie, comprenant une sonde (1) formée par un conduit allongé qui comporte un canal intérieur et des moyens de coupe configurés pour agir sur le tissu d'un patient, incluant un dispositif actionneur selon l'une quelconque des revendications 1 à 5, dans lequel les moyens de coupe peuvent être actionnés par le dispositif actionneur selon l'une quelconque des revendications 1 à 5, et dans lequel le dispositif actionneur est accouplé de manière amovible à ladite sonde.

7. Appareil médical (100) selon la revendication 6, dans lequel les moyens de coupe comprennent une lame de coupe située dans une fenêtre située dans la sonde (1), étant accouplée à un arbre d'entraînement qui s'étend le long du conduit allongé, l'arbre d'entraînement étant mobile sous l'action de l'arbre d'entraînement rotatif prévu dans le dispositif d'entraînement.

8. Appareil médical (100) selon la revendication 7, dans lequel la lame de coupe présente un bord qui est oblique par rapport à un bord de la fenêtre de coupe.
